(19) Europäisches Patentamt / European Patent Office / Office européen des brevets

(11) EP 1 677 749 B1

(12) **EUROPÄISCHE PATENTSCHRIFT**

(45) Veröffentlichungstag und Bekanntmachung des Hinweises auf die Patenterteilung:
**05.05.2010 Patentblatt 2010/18**

(21) Anmeldenummer: **04791212.6**

(22) Anmeldetag: **13.10.2004**

(51) Int Cl.:
***A61K 8/37*** *(2006.01)* ***A61Q 13/00*** *(2006.01)*
***C11D 3/50*** *(2006.01)* ***C11B 9/00*** *(2006.01)*

(86) Internationale Anmeldenummer:
**PCT/EP2004/052520**

(87) Internationale Veröffentlichungsnummer:
**WO 2005/037243 (28.04.2005 Gazette 2005/17)**

(54) **KURZKETTIGE ENOLESTER ALS RIECHSTOFF-PREKURSOREN**

SHORT-CHAIN ENOL ESTERS AS ODIFEROUS SUBSTANCE PRECURSORS

ENOLESTERS A CHAINE COURTE EN TANT QUE PRECURSEURS DE MATIERES ODORANTES

(84) Benannte Vertragsstaaten:
**AT BE BG CH CY CZ DE DK EE ES FI FR GB GR HU IE IT LI LU MC NL PL PT RO SE SI SK TR**

(30) Priorität: **16.10.2003 DE 10348062**

(43) Veröffentlichungstag der Anmeldung:
**12.07.2006 Patentblatt 2006/28**

(73) Patentinhaber: **Symrise GmbH & Co. KG**
**37603 Holzminden (DE)**

(72) Erfinder:
- **EH, Marcus**
**HOLZMINDEN 37603 (DE)**
- **PANTEN, Johannes**
**37671 Höxter (DE)**
- **BERTRAM, Heinz-Jürgen**
**37603 Holzminden (DE)**

(74) Vertreter: **Eisenführ, Speiser & Partner**
**Postfach 10 60 78**
**28060 Bremen (DE)**

(56) Entgegenhaltungen:
**EP-A- 0 035 183** **EP-A- 0 685 444**
**WO-A-95/04809** **CH-A- 629 655**
**DE-B- 1 109 678** **GB-A- 1 530 465**
**US-A- 4 933 321**

EP 1 677 749 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft ein Verfahren zur Freisetzung eines Riechstoffs sowie (a) Kosmetische, Wasch- und/oder Reinigungs-Formulierungen, die die Fragrance Precursor umfassen.

Die prinzipielle Vorgehensweise zur Parfümierung von Konsumartikeln ist die, dass das Riechstoffe enthaltene Parfümöl direkt mit dem Produkt vermischt wird. Als Problem tritt hierbei auf, dass zahlreiche Substanzen und hierbei besonders auch Aldehyde und teilweise auch Ketone instabil unter den gegebenen Bedingungen sind, was zur teilweisen oder vollständigen Zersetzung dieser Moleküle im Laufe der Lagerung führt. Die Konsequenz hieraus ist, dass alle Substanzen, die dem oben beschriebenen Problem unterliegen, im Endprodukt sensorisch nur noch schwach oder gar nicht mehr wahrnehmbar sind. Dies kann in Einzelfällen zu einer inakzeptablen Veränderung des Gesamtgeruchseindrucks der Komposition führen.

Aus US 5,649,979 sind Enolester vom Typ (II) bekannt,

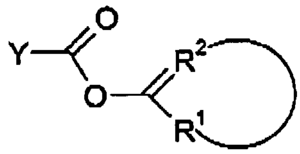 (II)

wobei Y einen unverzweigten oder verzweigten, gesättigten oder ungesättigten $C_7$ bis $C_{24}$ Rest darstellt, und einerseits $R^1$ = H und $R^2$ den Rest eines Riechstoffaldehydes $R^2CHO$ darstellt oder andererseits $R^1$ und $R^2$ Reste eines Riechstoffketons:

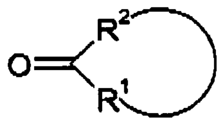

darstellen. Die beschriebenen Enolester setzen den Aldehyd oder das Keton langsam frei und werden für den Einsatz in Waschpulvern und Weichspülern beansprucht

Die US 6,207,857 offenbart Enolester der Formel (III)

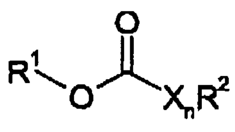 (III)

worin $R^1$ die Enolform eines näher definierten Aldehyds oder eines Ketons darstellt, X z.B. einen näher definierten Kohlenwasserstoffrest darstellt, $R^2$ z.B. einen näher definierten carbocyclischen oder heterocyclischen Rest oder COOY darstellt, wobei Y ein H-Atom, ein Metall oder $R^3$ ist, wobei $R^3$ der Rest eines Alkohols oder Phenols ist oder die gleiche Definition wie $R^1$ hat, und wobei n 0 oder 1 ist. Die Verbindungen der Formel (III) sollen nahezu geruchslos sein und unter Aktivierungsbedingungen eine oder mehrere Verbindungen freisetzen, die organoleptische und/oder antrimikrobielle Eigenschaften besitzen. Des Weiteren setzen die Fragrance Precursor der Formel (III) die aktiven Moleküle relativ langsam frei.

Weiterhin sind aus US 6,479,682 geschützte Hydroxyester der Formel (IV) bekannt

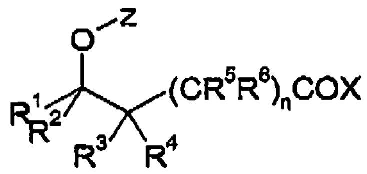

(IV)

worin die Substituenten näher definierte Bedeutungen besitzen. Die Verbindungen der Formel (IV) zerfallen in 2 Stufen, zuerst wird die "schutzgruppe" Z abgespalten und ein Hydroxyester gebildet, und in einem zweiten Schritt zyklisiert der Hydroxyester zu dem entsprechenden Lacton und spaltet dabei einen Alkohol, Aldehyd oder Keton ab. Die Verbindungen der Formel (IV) sollen nahezu geruchslos sein und auch hier erfolgt die Freisetzung der aktiven Moleküle langsam.

Die US 6,262,287 offenbart Siloxane der Formel (Va) und (Vb)

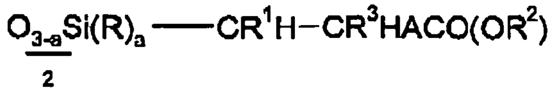

(Va)

oder

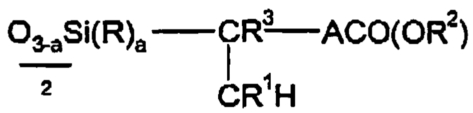

(Vb)

worin die Reste eine näher definierte Bedeutung haben. Die offenbarten Verbindungen der Formeln (Va) und (Vb) sind nahezu geruchslos und werden durch den Kontakt mit Haut oder durch Lipasen so gespalten, dass Riechstoffalkohole oder Aldehyde oder Ketone freigesetzt werden.

Der vorstehend gewürdigte Stand der Technik zeigt, dass bereits eine Reihe von Fragrance Precursoren bekannt sind, die mittels einer Esterfunktionalität die Enolform eines Aldehyds oder Ketons binden und nach Aktivierung den Aldehyd oder das Keton langsam, d.h. über einen Zeitraum von mehreren Strunden oder Tagen, freisetzen. Nachteiligerweise eignen sich die Fragrance Precursor, wie sie in der US 5,649,979, US 6,207,857, US 6,479,682 und US 6,262,287 offenbart sind, nicht für die nahezu spontane Freisetzung eines Aldehydes oder Ketons nach Aktivierung.

Es war deshalb die primäre Aufgabe der vorliegenden Erfindung, die Verwendung von Verbindungen als Fragrance Precursor anzugeben, die nach Einarbeitung in ein Produkt eine sehr viel höhere Lagerstabilität aufweisen, als die korrespondierenden (über ihre Enolform gebundenen) Aldehyde oder Ketone, und welche nach Aktivierung (Spaltung) die Aldehyde oder Ketone nahezu spontan freisetzen.

Erfindungsgemäß wird diese Aufgabe gelöst durch die Gegenstände des nachfolgenden Anspruchssatzes. Offenbart wird zudem eine Verwendung einer Verbindung der Formel I

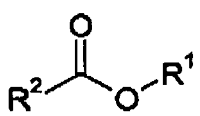

in der

$R^1$ der Rest (a) der Enolform eines Aldehyds mit 6 oder mehr C-Atomen oder (b) eines Ketons mit 10 oder mehr C-Atomen ist

und

$R^2$ eine (a) verzweigte oder unverzweigte $C_1$ bis $C_4$ Alkylgruppe oder (b) verzweigte oder unverzweigte $C_2$ bis $C_4$ Alkylengruppe ist,

als Riechstoff-Prekursor (fragrance precursor).

$R^2$ kann hierbei insbesondere sein:

(a) Methyl, Ethyl, n-Propyl, iso-Propyl, n-Butyl, sek.-Butyl, iso-Butyl, tert.-Butyl. Bevorzugt sind insoweit jedoch die Alkylreste Methyl, Ethyl, n-Propyl und iso-Butyl und besonders bevorzugt die Alkylreste Methyl, Ethyl und iso-Butyl.

(b) Ethenyl, Methylethenyl, 1-Propenyl, 2-Propenyl, 2-Methyl-1-propenyl, 1-Methyl-1-propenyl, 1-Butenyl, 3-Butenyl, Bevorzugt sind insoweit jedoch die Alkylenreste Ethenyl, Methylethenyl, 1-Propenyl, 2-Methyl-1-propenyl, 1-Methyl-1-propenyl und besonders bevorzugt die Alkylenreste Ethenyl, Methylethenyl und 1-Propenyl.

Überraschenderweise besitzen die erfindungsgemäß als Fragrance Precursor zu verwendenden Verbindungen der Formel (I) im Gegensatz zu den korrespondierenden Aldehyden oder Ketonen eine gute Lagerstabilität in (a) sauren, oxidativen Medien und (b) in alkalischen Medien mit einem Wassergehalt ≤ 10%. Die Lagerstabilität in sauren, oxidativen Medien ist insoweit überraschend, da in US 3923247 und Gerasimovich. T.B. et al. Natural'nykh Dushistykh Veshchestv, 1965, 38-42 beschrieben ist, dass Enolacetate in Gegenwart von 6N $H_2SO_4$ hydrolysiert werden.

Die Erfindung betrifft demnach ein Verfahren zur Freisetzung eines Riechstoffs, mit folgenden Schritten:

- Bereitstellen einer Verbindung der Formel I

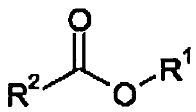

in der

$R^1$ der Rest (a) der Enolform eines Aldehyds mit 6 oder mehr C-Atomen oder (b) eines Ketons mit 10 oder mehr C-Atomen ist

und

$R^2$ eine (a) verzweigte oder unverzweigte $C_1$ bis $C_4$ Alkylgruppe oder (b) verzweigte oder unverzweigte $C_2$ bis $C_4$ Alkylengruppe ist,

- Herstellen einer Formulierung, die die Verbindung der Formel I und ein Medium umfasst, so dass die Verbindung der Formel I in der Formulierung stabil ist, wobei das Medium (a) sauer und oxidativ ist und einen Wassergehalt ≤ 10 Gew.-%, bezogen auf die Gesamtmasse des Mediums besitzt,

- Behandeln der Formulierung, so dass die Verbindung der Formel I zerfällt und den Riechstoff freisetzt.

Die Behandlung der Formulierung umfasst einen Schritt, in dem

- im Falle (a) der pH-Wert der Formulierung auf einen Wert ≥8,5 angehoben wird.

Auf diese Weise wird dafür gesorgt, dass die Verbindung der Formel I spontan zerfällt und den Riechstoff freisetzt. Der Riechstoff ist aus der Liste des Anspruchs 1 auszuwählen.

Die Formulierung selbst ist vorzugsweise

im Falle (a) aus der Gruppe ausgewählt, die besteht aus: Entwicklermasse für permanentes Haarfärbemittel, Dauerwellenfixierung, Bleichcreme, Aknecreme, Sanitärreiniger und Oberflächenreiniger.

Dieser Aspekt wird weiter unten im Detail erläutert.

Für den Fall (a) erfolgt die Aktivierung und somit nahezu spontane Zersetzung der als Fragrance Precursor zu verwendenden Verbindungen der Formel (I) und die damit einhergehende Freisetzung eines Aldehyds oder Ketons somit vorzugsweise durch die unmittelbare Anhebung des pH-Wertes in den alkalischen Bereich mit einem resultierenden pH-Wert ≥ 8.5.

Die vorliegende Erfindung betrifft auch kosmetische, Wasch- und/oder Reinigungs-Formulierungen, umfassend oder bestehend aus:

- einer Verbindung der Formel I

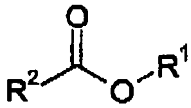

in der

$R^1$ der Rest (a) der Enolform eines Aldehyds mit 6 oder mehr C-Atomen oder (b) eines Ketons mit 10 oder mehr C-Atomen

und
$R^2$ eine (a) verzweigten oder unverzweigte $C_1$ bis $C_4$ Alkylgruppe oder (b) verzweigte oder unverzweigte $C_2$ bis $C_4$ Alkylengruppe ist
sowie

- einem Medium bestehend aus weiteren bzw. den weiteren Formulierungsbestandteilen, wobei das Medium (a) sauer und oxidativ ist und einen Wassergehalt ≤ 10 Gew.-%, bezogen auf die Gesamtmasse des Mediums besitzt,

wobei der Anteil der Verbindung der Formel I an der Formulierung geringer ist als 1 Gew.-%, bezogen auf die Gesamtmasse der Formulierung und wobei das Medium so ausgewählt ist, dass die Verbindung der Formel I in der Formulierung stabil ist.

Hinsichtlich der Verbindung der Formel I und ihrer bevorzugten Ausgestaltung gelten wieder die weiter oben gemachten Ausführungen.

Die Formulierung ist dann vorzugsweise im Falle (a) aus der Gruppe ausgewählt, die besteht aus: Entwicklermasse für permanentes Haarfärbemittel, Dauerwellenfixierung, Bleichcreme, Aknecreme, Sanitärreiniger und Oberflächenreiniger.

Vorteilhafterweise ist in einer erfindungsgemäßen Formulierung

(a) die Verbindung der Formel I in dem Medium dispergiert oder gelöst
und/oder

(b) die Verbindung der Formel I als Bestandteil eines Parfümöls (siehe dazu unten) eingesetzt, das in dem Medium dispergiert oder gelöst ist,

Dabei ist im Falle (b) das Parfümöl gegebenenfalls (i) an einem Trägerstoff adsorbiert, (ii) mikroverkapselt oder (iii) sprühgetrocknet oder es ist (iv) als Einschluss-Komplex oder (v) Extrusions-Produkt eingesetzt oder (vi) gecoatet.

Offenbart wird auch ein Parfümöl selbst, wobei dieses umfasst:

- eine Verbindung der Formel I

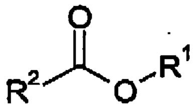

in der

$R^1$ der Rest (a) der Enolform eines Aldehyds mit 6 oder mehr C-Atomen oder (b) eines Ketons mit 10 oder mehr C-Atomen

und

$R^2$ eine (a) verzweigte oder unverzweigte $C_1$ bis $C_4$ Alkylgruppe oder (b) verzweigte oder unverzweigte $C_2$ bis $C_4$ Alkylengruppe ist

sowie

- einen oder mehrere Riechstoffe,

wobei der Anteil der Verbindungen der Formel I an dem Parfümöl mindestens 0,1 Gew-% beträgt, bezogen auf die Gesamtmasse des Parfümöls.

Das Parfümöl ist dabei gegebenenfalls (i) an einem Trägerstoff adsorbiert, (ii) mikroverkapselt oder (iii) sprühgetrocknet oder es ist (iv) als Einschluss-Komplex oder (v) Extrusions-Produkt eingesetzt oder (vi) gecoatet.

Weitere detaillierte Ausführungen zu den Parfümölen, die insbesondere zur Herstellung erfindungsgemäßer Formulierungen, zur Durchführung erfindungsgemäßer Verfahren und bei der Verwendung eingesetzt werden können, finden sich weiter unten. Es versteht sich, dass sämtliche im Rahmen des vorliegenden Textes gegebenen Erläuterungen zu den (bevorzugt) zu verwendenden Verbindungen der Formel I sämtliche Aspekte der Erfindung betreffen (Verwendung, Verfahren, Formulierung, Parfümöl etc.)

Aldehyde, die nach Spaltung einer erfindungsgemäß als Fragrance Precursor zu verwendenden Verbindung der Formel (I) freigesetzt werden, seien im folgenden genannt

Phenylacetaldehyd, p-Methylphenylacetaldehyd, p-Isopropylphenylacetaldehyd. Methylnonyl acetaldehyd, phenylpropanal, 3-(4-t-Butylphenyl)-2-methylpropanal (Lilial), 3-(4-t-Butylphenyl)-propanal (Bourgeonal), 3-(4-Methoxyphenyl)-2-methylpropanal (Canthoxal), 3-(4- Isopropylphenyl)-2-methylpropanal (Cymal), 3-(3,4-Methylendioxyphenyl)-2-methylpropanal (Helional), 3-(4-Ethylphenyl)-2,2-dimethylpropanal (Floralozone), Phenylbutanal, 3-Methyl-5-phenylpentanal, Hexanal, trans-2-Hexenal, cis-Hex-3-enal, Heptanal, cis-4-Heptenal, 2-Ethyl-2-heptenal, 2,6-Dimethyl-5-heptenal (Melonal), 2,4-Heptadienal, Octanal, 2-Octenal, cis-5-Octenal, 3,7-Dimethyloctanal, 3,7-Dimethyl-2,6-octadien-1-al, 3,7-Dimethyl-2,6-octadien-3-al, 3,7-Dimeihyl-6-octenal (Citronellal), 3,7-Dimethyl-7-hydroxyoctan-1-al (Hydroxy Citronellal), Nonanal, cis-6-Nonenal, 2,4-Nonadienal, 2,6-Nonadienal, Decanal, 2-Methyldecanal, 4-Decenal, 9-Decenal, 2,4-Decadienal, Undecanal, 2-Methyldecanal, 2-Methylundecanal, 2,6,90-Trimethys-9-undecenal (Adoxal), Undec-10-enylaldehyd, Undec-8-enanal, Dodecanal, Tridecanal, Tetradecanal, Anisaldehyd, Zimtaldehyd, α-Amylzimtaldehyd, α-Hexylzimtaldehyd, Methoxyzimtaldehyd, Isocyclocitral, Citronellyloxyacstaldehyd, Cortexaldehyd, Cuminaldehyd, Cyclamenaldehyd, Florhydral, Heliotropin, Hydratropaaldehyd, Vanillin, Ethylvanillin, Benzaldehyd, p-Methylbenzaldehyd, 3,4-Dimethoxybenzaldehyd, 3- und 4-(4-Hydroxy-4-methylpentyl)-3-cyclohexen-1-carboxaldehyd (Lyral), 2,4-Dimethyl-3-cyclohexen-1-carboxaldehyd (Triplal), l-Methyl-3-(4-methylpentyl)-3-cyclohexencarboxaldehyd (Vernaldehyd) oder p-Methylphenoxyacetaldehyd (Xi aldehyd) ist.

Ketone, die nach Spaltung einer erfindungsgemäß als Fragrance Precursor zu verwendenden Verbindung der Formel (I) freigesetzt werden, seien im folgenden genannt:

α-Damascon, β-Damascon, δ-Damascon, β-Damascenon, Muscon, 6,7-Dihydro-1,1,2,3,3-pentamethyl-4(5H)-indanon (Cashmeran), *cis*-Jasmon, Dihydrojasmon, α-Ionon, β-lonon, Dihydro-β-ionon, γ-Methylionon, α-*iso*-Methylionon, 4-(3,4-methylendioxyphenyl)butan-2-on, 4-(4-Hydroxyphenyl)butan-2-on, Methyl-β-naphthylketon, Methylcedrylketon, 6-Acetyl-1,1,2,4,4,7-hexamethyltetralin (Tonalid), l-Carvon, 5-Cyclohexadecen-1-on, Acetophenon, Decaton, p-Hydroxyphenylbutan-2-on, 2-[2-(4-Methyl-3-cyclohexenyl-1-yl)propyl]cyclopentan-2-on, 2-sec-Butylcyclohexanon, β-Dihydroionon, Allylionon, α-Iron, α-Ceton, α-Irison, Acetanisole, Geranylaceton, 1-(2-Methyl-5-isopropyl-2-cyclohexenyl)-1-propanon. Acetyldiisoamylen, Methylcyclocitron, 4-t-Pentylcyclohexanone, p-*t*-Butylcyclohexanon, *o-t* Butylcyclohexanon, Ethylamylketon, Ethylpentylketon, Menthon, Methyl-7,3-Dihydro-2H-1,5-benzodioxepin-3-on, Fenchon.

Aus den angegebenen Aldehyden und Ketonen ergeben sich selbstverständlich - nach Überführung in ihre jeweilige Enolform - die Reste $R^1$ in der Formel 1.

Die nahezu spontane Freisetzung eines Aldehyds oder Ketons nach Spaltung der erfindungsgemäßen als Fragrance Precursor zu benutzenden Verbindungen der Formel (I) kann zur Behandlung (z.B. Beduftung) von einer Fülle an Substraten, wie z.B. Haare, menschliche Haut, Wäsche und harte Oberflächen, benutzt werden.

Beispiele für Riechstoffe, mit denen sich die erfindungsgemäß als Fragrance Precursor zu verwendenden Verbindungen der Formel (I) vorteilhaft kombinieren lassen, finden sich z.B, in S. Arctander, Perfume and Flavor Materials, Vol. I und II, Montclair, N. J., 1969, Selbstverlag oder K. Bauer, D. Garbe und H, Surburg, Common Fragrance and Flavor Materials. 3rd. Ed., Wiley-VCH, Weinheim 1997.

Im einzelnen seien genannt:

Extrakte aus natürlichen Rohstoffen wie Etherische Öle, Concretes, Absolues, Resine, Resinoide, Balsame, Tinkturen wie z. B, Ambratinktur, Amyrisöl; Angelicasamenöl; Angelicawurzelöl; Anisöl; Baldrianöl; Basilikumöl; Baummoos - Absolue, Bayöl; Beifußöl; Benzoeresin; Bergamotteöl; Bienenwachs-Absolue; Birkenteeröl; Bittermandelöl, Bohnenkrautöl; Buccoblätteröl; Cabreuvaöl; Cadeöl; Calmusöl; Campheröl; Canangaöl; Cardamomenöl; Cascarillaöl; Cassiaöl: Cassie-Absolue; Castoreum-absolue; Cedemblätteröl; Cedemholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue: Eichenmoos-Absolue; Elemiöl; Estragonöl: Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Klefemadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenbloten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;

Einzel-Riechstoffe aus der Gruppe der Kohlenwasserstoffe, wie z.B. 3-Caren; α Pinen; β-Pinen; α-Terpinen; γ-Terpinen; p-Cymol; Bisabolen; Camphen; Caryophyllen; Cedren; Famesen; Limonen; Longifolen; Myrcen; Ocimen; Valencen; (E,Z)-1,3,5-Undecatrien;

der aliphatischen Alkohole wie z. B. Hexanol; Octanol; 3-Octanol; 2,6-Dimethylheptanol; 2-Methylheptartol, 2-Methyloctanol; (E)-2-Hexenol; (E)- und (Z)-3-Hexenol: 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol: 10-Undecenol; 4-Methyl-3-decen-5-ol; der aliphatischen Aldehyde und deren 1,4-Dioxacycloalken-2-one wie z. B. Hexanal; Heptanal; Octanal; Nonanal: Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal: 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;

der aliphatischen Ketone und deren Oxime wie z.B, 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;

der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;

der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetet; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-Isoamyloxyacetat; Methyl-3,7-dimethyl-2,6-octadienoat;

der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-01; 3,7-Dimethyl-4,6-octadien-3-ol; 3,7-Dimethyl-1,5,7-octatrlen-3-ol 2,6-Dimethyl-2,5,7-octatrien-1-ol; sowie deren Formiate, Acetate, Propionate, Isobutyrate, Butyrate, Isovalerfanate, Pentanoate, Hexanoate, Crotonate, Tiglinate, 3-Methyl-2-butenoate;

der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;

der cyclischen Terpenalkohole wie z.B. Menthol; Isopulegol; alpha-Terpineol; Terpinenol-4; Menthan-8-ol, Menthan-1-ol; Menthan-7-ol; Borneol; Isoborneol; Linalooloxid; Nopol; Cedrol; Ambrinol; Vetiverol; Guajol, sowie deren Formiate, Acetate, Propionate, Isobutyrate, Butyrate, Isovalerianate, Pentanoate, Hexanoate, Crotonate, Tiglinate, 3-Methyl-2-butenoate;

der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);

der cyclischen Alkohole wie z.B. 4-tert-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-isobutyl-4-methyltetrahydro-2H-pyran-4-ol;

der cycloaliphatischen Alkohole wie z.B. alpha,3,3-Trimethylcyclohexylmethanol; 2-Methyl-4-(2,2,3-trimethyl-3-cyclopent-1-yl)butanol; 2,Methyl-4-(2,2,3-trimemyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Mothyl-5-(2,2,3-trimethyl-3-cyclopent-1-yl)-4-penten-2-ol; 3,3-Dlmethyl-5-(2,2,3-trimethyl-3--cyclopent-1-yl)-4-penten-2-ol; 1-(2,2,6-Trimethylcyclohexyl)pentan-3-ol; 1-(2,2,6-Trimethylcyclohexyl)hexan-3-ol;

der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;

der cyclischen Ketone wie z.B. 4-tert-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert-Pentylcyclohexanon; 5-Cyrlohexadecan-1-on; 6,7-Dihydro-1,1,2.3.3-pentamethyl-4(5H)-indanon; 5-Cyclohexadecen-1-on; 8-Cyclohexadecen-1-on; 9-Cycloheptadecen-1-on; Cyclopentadecanon;

der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;

der cycloaliphatischen Ketone wie z. B. 1-(3,3-Dimethyloyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cylcododecatrienylketon: tert.-Butyl-(2,4-dimethyl-3-cyclohexen-1-yl)keton;

der Ester cyclischer Alkohole wie z.B. 2-terl-Butylcyclohexylacetat 4-tert Butylcyclohexylacetat; 2-tert-Pentylcyclohewlacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;

der Ester cycloaliphatischer Carbonsäuren wie z. B. Allyl-3-cyclohexylproplonat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;

der aromatischen Kohlenwasserstoffe wie z. B. Styrol und Diphenylmethan,

der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phertylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;

der Ester von araliphatischen Alkoholen mit aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovaaerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether, 2-Phenylethylisoamylether, 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydro-indeno [1,2-d]-m-dioxin; 4,4a,5,9b-Tetrahydro-2,4-dimethylindeno[1,2-d]-m-dioxin;

der aromatischen oder araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2.2-dimethylpropanal; 2-Methyl-3-(4-isopropylphenyl)propanal; 2-Methyl-3-(4-tert-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd: 2-Methyl-3-(4-methoxyphenyl)propanal; 2-Methyl-3-(4-methylendioxyphenyl)propanal;

der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methytethyl)-1H-5-indenyl]ethanon; 5',6',7',8'-Tetr-ahydro-3',5',5',6',8',8'-hexamethyl-2-acetonaphthon;

der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;

der stickstoffhattigen aromatischen Verbindungen wie z.B. 2,4,6-Trinitro-1,3-dimethyl-5-tert-butylbenzol; 3,5-Dinitro-2,6-dimethyl-4-tert-butylacetophenon; Zimtsäurenitril; 5-Phenyl-3-methyl-2-pentensäurenitril; 5-Phenyl-3-methylpentansäurenitril; Methylanthranilat; Methy-N-methylanthranliat; Schiff'sche Basen von Methylanthranilat mit 7-Hydroxy-3,7-dimethyioctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;

der Phenole, Phenylether oder Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenyl; 2-Ethoxy-5-(1-propenyl)phenol; p-Kresylphenylacetat;

der heterocyclischen Verbindungen wie z.B. 2,5-Dimethyl-4-hydroxy-2H-furan-3-on; 2-Etthyl-4-hydroxy-5-methyl-2H-furan-3-on; 3-Hydroxy-2-methyl-4H-pyran-4-on; 2-Ethyl-3-hydroxy-4H-pyran-4-on;

der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Parfümöle, die einen oder mehrere als Fragrance Precursor zu verwendenden Verbindungen der Formel (I) enthalten, können in flüssiger Form, unverdünnt oder mit einem Lösungsmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können Parfümöle, die eine oder mehrere als Fragrance Precursor zu verwendende Verbindungen der Formel (I) enthalten, an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Parfümöle, die eine oder mehrere als Fragrance Precursor zu verwendende Verbindungen der Formel (I) enthalten, können auch mikroverkapselt, sprühgetrocknet, als Einschluss-Komplex oder als Extrusions-Produkt vorliegen und in dieser Form dem zu parfümierenden Produkt hinzugefügt werden.

Gegebenenfalls können die Eigenschaften der derart modifizierten Parfümöle durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmsterialien z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine. Dextrin und pflanzliche Gummen verwendet werden können. Einschlusskomplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, gegebenenfalls in einem geeigneten Lösungsmittel, z.B. Isopropanol, erfolgen.

In Parfümkompositionen beträgt die eingesetzte Menge der erfindungsgemäßen als Fragrance Precursor zu benutzenden Verbindungen der Formel (I) 0,01 bis 75 Gew.%, vorzugsweise 0,05 bis 50 Gew.-%, besonders bevorzugt ist eine Einsatzmenge von 0,5 bis 20 %, bezogen auf das gesamte Parfümöl.

Parfümöle, die die als Fragrance Precursor zu benutzenden Verbindungen der Formel (I) enthalten, können in konzentrierter Form, in Lösungen oder in oben beschriebener modifizierter Form für die Herstellung von kosmetischen Pflege-Produkten verwendet werden. Hierbei insbesondere für Haarpflege- oder Waschprodukte, in denen die Stabilität von Aldehyden oder Ketonen gering ist und bei denen (a) durch die unmittelbare Anhebung des pH-Wertes von einem sauren pH-Wert in den alkalischen Bereich mit einem resultierenden pH-Wert $\geq 8.5$ oder (b) durch Zugabe von Wasser, eine Aktivierung und somit eine nahezu spontane Freisetzung des Aldehyds oder Ketons bewirkt wird. Als Beispiele seien hier genannt: Körperpflegemittel wie z.B. feste und flüssige Seife, Bleichcremes, Aknecremes, Haarpflegeprodukte wie z.B. festigende Haarlotionen, permanente Haarfärbemittel, Deodorantien und Antiperspirantien wie z.B. Deo- und Antiperspirantsticks.

Bevorzugt können Parfümöle, die die als Fragrance Precursor zu benutzenden Verbindungen der Formel (I) enthalten, in konzentrierter Form, in Lösungen oder in oben beschriebener modifizierter Form für die Herstellung von Haarpflegeprodukten und Körperpflegemitteln und hierbei insbesondere für die Herstellung von permanenten Haarfärbemitteln eingesetzt werden.

Weiterhin können Parfümöle, die die als Fragrance Precursor zu benutzenden Verbindungen der Formel (I) enthalten, in konzentrierter Form, in Lösungen oder in oben beschriebener modifizierter Form für die Herstellung von z.B. Haushaltprodukten, wie Fußbodenreinigem, Fensterglasreiniger, Bad- und Sanitärreiniger, festen und flüssigen WC-Reiniger, flüssigen Waschmittel, pulverförmigen Waschmittel, Wäschevorbehandlungsmittel wie Bleichmittel, Einweichmittel und Fleckenentferner, Waschtabletten, Desinfektionsmittel, Oberflächendesinfektionsmittel eingesetzt werden.

Bevorzugt können Parfümöle, die die als Fragrance Precursor zu benutzenden Verbindungen der Formel (I) enthalten, in konzentrierter Form, in Lösungen oder in oben beschriebener modifizierter Form für die Herstellung von flüssigen Waschmitteln eingesetzt werden.

Die als Fragrance Precursor zu verwendenden Verbindungen der Formel (I), können nach dem Fachmann wohlvertrauten Methoden hergestellt werden. Die Enolester der Formel (I) worin $R^2$ eine (a) verzweigte oder unverzweigte $C_1$ bis $C_3$ Alkylgruppe oder (b) verzweigte oder unverzweigte $C_2$ bis $C_3$ Alkylengruppe bedeutet, werden nach der Vorschrift aus D.P. Simmons et al., Helv. Chim. Acta 71, 1000 (1988) hergestellt. Die Enolester der Formel (I) worin $R^2$ eine (a) verzweigte oder unverzweigte $C_4$ Alkylgruppe oder (b) verzweigte oder unverzweigte $C_4$ Alkylengruppe bedeutet, werden nach der Vorschrift aus P. Duhamel et al., J. Chem. Soc. Perkin Trans. 1, 1993, 2509 hergestellt.

Die folgenden, nicht limitierenden Beispiele erläutern die Erfindung.

**Beispiel 1:**

**Herstellung von (E/Z)-Essigsäure-(2,4-dimethylcyclohex-3-enyliden)methylester**

Man legt 2,4-Dimethylcyclohex-3-en-1-carbaldehyd (13.82 g, 100.0 mmol), Natriumacetat (1.54 g, 18.5 mmol) und Triethylamin (21.27 g, 210.0 mmol) in Essigsäureanhydrid (150 ml) vor und erhitzt für 6 Stunden auf 120°C. Nach beendeter Reaktion (DC-Kontrolle) lässt man abkühlen, gießt die Reaktionslösung in Eiswasser (100 ml) und extrahiert die wässrige Phase mit Ether (150 ml) und Cyclohexan (150 ml). Die vereinigten organischen Phasen werden je 1x mit 2M NaOH (100 ml) und Wasser (100 ml) gewaschen, anschließend über $Na_2SO_4$ getrocknet, abfiltriert und einrotiert.

Nach fraktionierter Destillation (60.0-61.6 °C, 0.25 mbar) erhält man 17.5 g (E/Z)-Essigsäure-(2,4-dimethylcyclohex-3-enyliden)methylester als farbloses Öl,

E/Z-Isomerenverhältnis = 1:1.

Spektroskopische Daten entsprechen dem E-Isomeren:

$^1$H-NMR (400 MHz, $CDCl_3$): δ (ppm) =1.10 (d, 7.1 Hz, 3H), 1.64-1.67 (m, 3H), 1.97-2.05 (m, 2H), 2.14 (s, 3H), 2.43 (ddd, J = 0.6, 7.2, 13.2 Hz, 1H), 2.49 (ddd, J = 0.9, 5.7, 13.2 Hz, 1H), 3.18-3.28 (m, 1H), 5.26-5.31 (m, 1H), 7.00 (q, 1.1 Hz, 1H).

$^{13}$C-NMR (100 MHz, $CDCl_3$): δ (ppm) = 20.7, 20.8, 21.4, 23.3, 30.9, 33.4, 125.5, 126.7. 128.0, 133.3, 168.4.

**<u>Beispiel 2:</u>**

**Herstellung von (E/Z)-Isobuttersäure-(2,4-dimethylcyclohex-3-enyllden)methylester**

Die Herstellung von (E/Z)-2-Methylpropionsäure-(2,4-dimethylcyclohex-3-enyliden)methylester erfolgt analog Beispiel 1, wobei Isobuttersäureanhydrid anstatt Essigsäureanhydrid eingesetzt wurde.

E/Z-Isomerenverhältnis = 1:1.

Spektroskopische Daten entsprechen dem E-Isomeren:

$^1$H-NMR (400 MHz, $CDCl_3$): δ (ppm) = 1.07 (d, 7.1 Hz, 3H), 1.22 (d, J = 7.0 Hz, 6H), 1.64-1.67 (m , 3H), 1.98-2.05 (m, 2H), 2.36 (ddd, J = 0.7, 7.3. 13.4 Hz, 1H), 2.48 (ddd, J = 0.9, 6.1, 13.4 Hz, 1H), 2.62 (sep, J = 7.0 Hz, 1H), 3.20-3.26 (m, 1H), 5.26-5.31 (m, 1H), 7.00 (q, 1.1 Hz, 1H).

$^{13}$C-NMR (100 MHz, $CDCl_3$): δ (ppm) = 18.8 (2C), 20.7, 23.5, 23.8, 29.9, 31.3, 34.0, 125.5, 126,6, 127.5, 133.3, 174.1.

**<u>Beispiel 3:</u>**

**Herstellung von (E/Z)-Pivalinsäure-(2,4-dimethylcyclohex-3-enyliden)methylester**

Man legt (E/Z)-Essigsäure-(2,4-dimethylcyclohex-3-enyliden)methylester (26.27 g, 145 mmol) in THF (200 ml) vor, kühlt auf -70°C ab, und gibt Kaliumtert-butanolat (24.75 g, 220 mmol), gelöst in THF (100 ml), hinzu. Jetzt lässt man 60 Minuten bei -70°C nachrühren, bevor man Pivalinsäurechlorid (26.57 g, 220 mmol), gelöst in THF (60 ml), zugibt und anschließend noch weitere 120 Minuten nachrühren lässt. Nach beendeter Reaktion (DC-Kontrolle) gießt man die Reaktionslösung auf ges. $NaHCO_3$-Lösung (250 ml), trennt die Phasen und extrahiert die wässrige Phase noch 2x mit Ether (250 ml). Die vereinigten organischen Phasen werden über $Na_2SO_4$ getrocknet, abfiltriert und einrotiert. Das erhaltene Rohprodukt wird mittels Flashchromatograhie (Cyclohexan/EtOAc = 60:1, $R_f$ = 0.23) gereinigt, und man erhält 24.50 g eines farblosen Öls.

E/Z-Isomerenverhältnis = 2:1.

Spektroskopische Daten entsprechen dem E-Isomeren:

$^1$H-NMR (400 MHz, $CDCl_3$): δ (ppm) = 1.08 (d, 7.1 Hz, 3H), 1.25 (s, 9H), 1.66-1.68 (m , 3H), 1.97-2.05 (m, 2H), 2.38 (ddd, J = 0.6, 7.2, 13.2 Hz, 1H), 2.49 (ddd, J = 0.9, 5.7, 13.2 Hz, 1H), 3.19-3.28 (m, 1H), 5.27-5.31 (m, 1H), 6.90 (q, 2.2 Hz, 1H).

$^{13}$C-NMR (100 MHz, $CDCl_3$): δ (ppm) = 21.3, 23.5, 23.8, 27.1 (3C), 30.0, 31.2, 38.8, 125.5, 126.5, 127.7, 133.4, 175.5.

**<u>Beispiel 4:</u>**

**Herstellung von (1E/Z)-Essigsäuredec-1-enylester**

Die Synthese erfolgt analog Beispiel 1, wobei Decanal anstatt 2,4-Dimethylcyclohex-3-en-1-carbaldehyd eingesetzt wurde. Siehe auch P.Z, Bedoukian, J. Am. Chem. Soc. 79, 889-892, (1957).

**Beispiel 5:**

**Herstellung von (1E/Z)-Isobuttersäuredec-1-enylester**

Die Synthese erfolgt analog Beispiel 1, wobei Decanal anstatt 2,4-Dimethylcyclohex-3-en-1-carbaldehyd und Isobuttersäureanhydrid anstatt Essigsäureanhydrid eingesetzt wurde.

**Beispiel 6:**

**Herstellung von (1E/Z)-Pivalinsäuredec-1-enylester**

Die Synthese erfolgt analog Beispiel 3, wobei (1E/Z)-Essigsäuredec-1-enylester anstatt (E/Z)Essigsäure-(2,4-dimethylcyclohex-3-enyliden)methylester eingesetzt wurde.

**Beispiel 7:**

**Herstellung von (1E/Z)-Essigsäure-3-methyl-5-phenylpent-1-enylester**

Die Synthese erfolgt analog Beispiel 1, wobei 3-Methyl-5-phenylpentanal anstatt 2,4-Dimethylcyclohex-3-en-1-carbaldehyd eingesetzt wurde.

**Beispiel 8:**

**Herstellung von (1E/Z)-Isobuttersäure-3-methyl-5-phenylpent-1-enylester**

Die Synthese erfolgt analog Beispiel 1, wobei 3-Methyl-5-phenylpentanal anstatt 2,4-Dimethylcyclohex-3-en-1-carbaldehyd und Isabuttersäureanhydrid anstatt Essigsäureanhydrid eingesetzt wurde.

**Beispiel 9:**

**Herstellung von (1E/Z)-Pivalinsäure-3-methyl-5-phenylpent-1-enylester**

Die Synthese erfolgt analog Beispiel 3, wobei (1E/Z)-Essigsäure-3-methyl-5-phenylpent-1-enylester anstatt (E/Z)-Essigsäure-(2,4-dimethylcyclohex-3-enyliden)methylester eingesetzt wurde.

**Beispiel 10:**

**Herstellung von (1E/Z)-Essigsäure-3-(4-tert-butylphenyl)-2-methylprop-1-enylester**

Die Synthese erfolgt analog Beispiel 1, wobei 3-(4-tert-Butylphenyl)-2-methylpropanal anstatt 2,4-Dimethylcyclohex-3-en-1-carbaldehyd eingesetzt wurde. Siehe auch JP 5514137 A1.

**Beispiel 11:**

**Herstellung von (1E/Z)-Isobuttersäure-3-(4-tert-butylphenyl)-2-methylprop-1-enylester**

Die Synthese erfolgt analog Beispiel 1, wobei 3-(4-tert-Butylphenyl)-2-methylpropanal anstatt 2,4-Dimethylcyclohex-3-en-1-carbaldehyd und Isobuttersäursanhydrid anstatt Essigsäureanhydrid eingesetzt wurde.

**Beispiel 12:**

**Herstellung von (1E/Z)-Pivalinsäure-3-(4-tert-butylphenyl)-2-methylprop-1-enylsster**

Die Synthese erfolgt analog Beispiel 3, wobei (1E/Z)-Essigsäure-3-(4-tert-butylphenyl)-2-methylprop-1-enylester anstatt (E/Z)-Essigsäure-(2,4-dimethylcyclohex-3-enyliden)methylester eingesetzt wurde.

**Beispiel 13:**

**Herstellung von (1E/Z)-Essigsäure-3-(1,3-benzodioxol-5-yl)-2-methylprop-1-enylester**

Die Synthese erfolgt analog Beispiel 1, wobei 3-(1,3-Benzodioxol-5-yl)-2-methylpropanal anstatt 2,4-Dimethylcyclohex-3-en-1-carbaldehyd eingesetzt wurde.

**Beispiel 14:**

**Herstellung von (1E/Z)-Isobuttersäure-3-(1,3-benzodioxol-5-yl)-2-methylprop-1-enylester**

Die Synthese erfolgt analog Beispiel 1, wobei 3-(1,3-Benzodioxot-5-yl)-2-methylpropanal anstatt 2,4-Dimemylcyclohex-3-en-l-carbaldehyd und Isobuttersäureanhydrid anstatt Essigsäureanhydrid eingesetzt wurde.

**Beispiel 15:**

**Herstellung von (1E/Z)-Pivalinsäure-3-(1,3-benzodioxol-5-yl)-2-methylprop-1-enylester**

Die Synthese erfolgt analog Beispiel 3, wobei (1E/Z)-Essigsäure-3-(1,3-benzodioxol-5-yl)2-methylprop-1-enylester anstatt (E/Z)-Essigsäure-(2,4-dimethylcyclohex-3-enyliden)methylester eingesetzt wurde.

**Beispiel 16:**

**Herstellung von (1E/Z)-Essigsäure-dodec-1-enylester**

Die Synthese erfolgt analog Beispiel 1, wobei Dodecanal anstatt 2,4-Dimethylcyclohex-3-en-1-carbaldehyd eingesetzt wurde. Siehe auch P.Z. Bedoukian, J. Am. Chem. Soc. 79, 889-892, (1957).

**Beispiel 17:**

**Herstellung von (1E/Z)-Isobuttersäure-dodec-1-enylester**

Die Synthese erfolgt analog Beispiel 1. wobei Dodecanal anstatt 2,4-Dimethylcyclohex-3-en-1-carbaldehyd und Isobuttersäureanhydrid anstatt Essigsäureanhydrid eingesetzt wurde.

**Beispiel 18:**

**Herstellung von (1E/Z)-Pivalinsäure-dodec-1-enylester**

Die Synthese erfolgt analog Beispiel 3, wobei (1E/Z)-Essigsäure-dodec-1-enylester anstatt (E/Z)-Essigsäure-(2,4-dimethylcyclohex-3-enyliden)methylester eingesetzt wurde.

**Beispiel 19:**

**Herstellung von (1E/Z)-Essigsäure-2,6-dimethylhepta-1,5-dienylester**

Die Synthese erfolgt analog Beispiel 1, wobei 2,6-Dimethylhept-5-enal anstatt 2,4-Dimethylcyclohex-3-en-1-carbaldehyd eingesetzt wurde. Siehe auch JP 55015433 B4.

**Beispiel 20:**

**Herstellung von (1E/Z)-Isobuttersäure-2,6-dimethylhepta-1,5-dienylester**

Die Synthese erfolgt analog Beispiel 1, wobei 2,6-Dimethylhept-5-enal anstatt 2,4-Dimethylcyclohex-3-en-1-carbaldehyd und Isobuttersäureanhydrid anstatt Essigsäureanhydrid eingesetzt wurde,

**Beispiel 21:**

**Herstellung von (1E/Z)-Pivalinsäure-2,6-dimethylhepta-1,5-dienylester**

Die Synthese erfolgt analog Beispiel 3, wobei (1E/Z)-Essigsäure-2,6-dimethylhepta-1,5-dienylester anstatt (E/Z)-Essigsäure-(2,4-imethylcyclohex-3-enyliden)methylester eingesetzt wurde.

**Beispiel 22:**

**Herstellung von (1E/Z,3E/Z)-Essigsäure-hexa-1,3-dienylester**

Die Synthese erfolgt analog Beispiel 1, wobei (3E)-Hex-3-enal anstatt 2,4-Dimethylcyclohex-3-en-1-carbaldehyd eingesetzt wurde. Siehe auch B.M. Trost et al. J. Am. Chem. Soc. 100, 3930-3931, (1978).

**Beispiel 23:**

**Herstellung von (1E/Z,3E/Z)-Isobuttersäure-hexa-1,3-dienylester**

Die Synthese erfolgt analog Beispiel 1, wobei (3E)-Hex-3-enal anstatt 2,4-Dimethylcyclohex-3-en-1-carbaldehyd und Isobuttersäureanhydrid anstatt Essigsäureanhydrid eingesetzt wurde.

**Beispiel 24:**

**Herstellung von (1E/Z,3E/Z)-Pivalinsäure-hexa-1,3-dienylester**

Die Synthese erfolgt analog Beispiel 3, wobei (1E/Z,3E/Z)-Essigsäure-hexa-1,3-dienylester anstatt (E/Z)-Essigsäure-(2,4-dimethylcyclohex-3-enyliden)methylester eingesetzt wurde.

**Beispiel 25:**

**Herstellung von (1E/Z,5Z)-Essigsäure-octa-1,5-dienylester**

Die Synthese erfolgt analog Beispiel 1. wobei (5Z)-Oct-5-enal anstatt 2,4-Dimethylcyclohex-3-en-1-carbaldehyd eingesetzt wurde.

**Beispiel 26:**

**Herstellung von (1E/Z,6Z)-Essigsäure-nona-1,6-dienylester**

Die Synthese erfolgt analog Beispiel 1, wobei (6Z)-Non-6-enal anstatt 2,4-Dimethylcyclohex-3-en-1-carbaldehyd eingesetzt wurde.

**Beispiel 27:**

**Herstellung von (1E/Z)-Essigsäure-3-(4-isopropylphenyl)-2-methylprop-1-enylester**

Die Synthese erfolgt analog Beispiel 1, wobei 3-(4-Isopropylphenyl)-2-methylpropanal anstatt 2,4-Dimethylcyclohex-3-en-1-carbaldehyd eingesetzt wurde. Siehe auch US 3023247.

**Beispiel 28:**

**Herstellung von (1E/Z)-Essigsäure-2,6,10-trimethylundeca-1,9-dienylester**

Die Synthese erfolgt analog Beispiel 1, wobei 2,6,10-Trimethylundec-9-enal anstatt 2,4-Dimethylcyclohex-3-en-1-carbaldehyd eingesetzt wurde.

Die erfindungsgemäß als Fragrance Precursor zu verwendenden Verbindungen der Formel (I) wurden in zahlreiche Verbraucherprodukte eingearbeitet und deren anwendungstechnische Eigenschaften mit verschiedenen Methoden untersucht. Bei der Herstellung der Formulierungen für die Verbraucherprodukte wurden molare Äquivalente der

Aldehyde oder Ketone einerseits in Form der Enolester bzw. andererseits in Form der freien Aldehyde oder Ketone eingesetzt, um eine Vergleichbarkeit zu gewährleisten.

Methode 1: Lagerstabilität

Die Lagerstabilität eines Riechstoffes bzw. eines Fragrance Precursors wird durch die prozentuale Menge des nach Lagerung noch vorhandenen Stoffes definiert.

$$\frac{\text{Menge nach der Lagerung}}{\text{Menge vor der Lagerung}} * 100\% = \text{Lagerstabilität [\%]}$$

Zur Bestimmung und zum Vergleich der Lagerstabilität werden sowohl der Fragrance Precursor und die korrespondierenden Aldehyde oder Ketone in separate Muster der gleichen Formulierung eines Verbraucherproduktes wie z.B. Haarfärbemittel oder Seife eingearbeitet. Anschließend werden die separaten Muster in Portionen geteilt. Die eine Portion der Muster wird unverzüglich einer geeigneten Extraktion und einer analytischen Messung unterzogen, um die Menge an Fragrance Precursor bzw. Aldehyd oder Keton vor der Lagerung zu bestimmen. Bei der analytischen Untersuchung durch z.B.

Gaschromatographie wird zur Quantifizierung ein geeigneter Standard verwendet. Die zweite Portion wird einer Lagerung bei erhöhter Temperatur für eine definierte Zeit unterzogen und anschließend mit den gleichen Methoden extrahiert und quantifiziert.

**Beispiel 29: Permanentes Haarfärbemittel**

(a) Stabilität in der Entwicklermasse:

Die Formulierung der Entwicklermasse enthält typischerweise Wasser, Wasserstoffperoxid, Säuren wie z.B. Phosphorsäure, Citronensäure usw., Verdicker, Emulgatoren, Konservierungsmittel, Komplexbildner, Silikone, Lösungsmittel und weitere Hilfsstoffe.

Zu Chargen der Entwicklerformulierung werden die Fragrance Precursoren (siehe Tabelle 1) in einer Dosierung von 1% gegeben, und für einen Monat bei 40°C gelagert.

**Tabelle 1:_Lagerstabilität von Fragrance Precursoren in Entwicklermasse**

| Fragrance Precursor | Stabilität [%] 0 Tage | Stabilität [%] 13 Tage | Stabilität [%] 28 Tage |
|---|---|---|---|
| (1E/Z)-Essigsäuredec-1-enylester | 100 | 100 | 98 |
| (1E/Z)-Essigsäure-3-methyl-5-phenylpent-1-enylester | 100 | 100 | 100 |
| (1E/Z)-Essigsäure-3-(4-tert-butylphenyl)-2-methylprop-1-enylester | 100 | 100 | 97 |
| (E/Z)-Isobuttersäure-(2,4-dimethylcyclohex-3-enyliden)methylester | 100 | 100 | 100 |
| (1E/Z)-Isobuttersäure-3-(1,3-benzodioxol-5-yl)-2-methylprop-1-enylester | 100 | 100 | 100 |
| (1E/Z)-Isobuttersäure-dodec-1-enylester | 100 | 100 | 100 |
| (E/Z)-Pivalinsäure-(2,4-dimethylcyclohex-3-enyliden)methylester | 100 | 100 | 100 |
| (1E/Z)-Pivalinsäure-3-(4-tert-butylphenyl)-2-methylprop-1-enylester | 100 | 100 | 100 |
| (1E/Z)-Pivalinsäure-dodec-1-enylester | 100 | 100 | 100 |

Die Fragrance Precursor sind über den einmonatigen Zeitraum farblich, geruchlich und analytisch stabil.

(b) Freisetzungrate während der Haarfärbung:

Zur Bestimmung der Hydrolysegeschwindigkeit wurden verschiedene Fragrance Precursor (siehe Tabelle 2) in einer Konzentration von jeweils 0,3% zu der Entwicklermasse gegeben. Anschließend fügt man zu der Entwicklermasse im Verhältnis 1:1 die ammoniakalische Färbemasse, welche sich aus 2 bis 16 % Ammoniak und/oder Ersatzstoffen wie z,B, Alkanolamine, insbesondere Monoethanolamin, Wasser, Verdicker, Emulgator, Konsistenzbildner, Reaktivfarbstoffe, Lösungsmittel, Komplexbildner, Stabilisatoren und Konservierungsmittel zusammensetzt, hinzu. Jetzt werden in definierten zeitlichen Abständen Proben aus der Haarfärbemasse entnommen, die Proben werden sofort neutralisiert, mit Lösungsmittel extrahiert und der Gehalt an Fragrance Precursor und freigesetztem Aldehyd oder Keton mittels Gaschromatographie unter Verwendung eines internen und externen Standards bestimmt.

**Tabelle 2: Analytisch bestimmte Aldehydfreisetzung während der Haarfärbung**

| Aldehyde | Maximale Aldehydkonzentration in [%] nach | | | | |
|---|---|---|---|---|---|
| | 1 Min. | 5 Min. | 10 Min. | 20 Min. | 30 Min. |
| 2,4-Di-methylcyclohex-3-en-1-carbaldehyd freigesetzt aus Bespiel 1 | 65 | 100 | 100 | 75 | 70 |
| 3-(4-tert-Butylphenyl)-2-methylpropanal freigesetzt aus Beispiel 10 | 29 | 91 | 100 | 83 | 80 |
| 3-Methyl-5-phenylpentanal freigesetzt aus Beispiel 7 | 90 | 100 | 87 | 87 | 84 |
| Decanal freigesetzt aus Beispiel 4 | 13 | 92 | 100 | 92 | 84 |
| 3-(1,3-Benzodioxol-5-yl)-2-methylpropanal freigesetzt aus Beispiel 13 | 29 | 100 | 91 | 85 | 82 |

Die Fragrance Precursor in der Formulierung für Haarfärbung zeigten nach Vereinigung der Entwicklermasse mit der ammoniakalischen Haarfärbelösurlg eine nahezu spontane Hydrolyse zu den korrespondierenden Aldehyden. Bereits nach 5 Minuten waren aus allen Fragrance Precursoren nahezu 100% der maximalen Aldehydkonzentration entstanden.

(c) Geruchliche Beurteilung:

Die geruchliche Stärke der einzelnen freigesetzten Aldehyde wurde sensorisch von der Haarsträhne bestimmt. Die Skala der sensorischen Intensität reicht von 1,0 = geruchslos bis zu 9,0 = sehr stark. Die Entwickler wurden analog dem unter (b) beschriebenen Experiment hergestellt, nach Zusammenführung der einzelnen Entwickler mit der ammoniakalischen Färbemasse wurde die resultierende Haarfärbemasse auf die Haarsträhnen aufgetragen und von einem Panel (5 Personen) die geruchliche Intensität der freigesetzten Aldehyde nach bestimmten Zeitintervallen bestimmt.

**Tabelle 3: Sensorisch bestimmte Aldehydintensität während der Haarfärbung**

| Aldehyde | Aldehydintensität nach | | | | | | |
|---|---|---|---|---|---|---|---|
| | 2 Min. | 4 Min. | 6 Min. | 8 Min. | 10 Min. | 15 Min. | 30 Min. |
| 2,6-Dimethylhept-5-enal freigesetzt aus Beispiel 19 | 5.5 | 5.7 | 5.7 | 5.5 | 5.3 | 5.3 | 5.0 |
| Dodecanal freigesetzt aus Beispiel 16 | 5.2 | 5.0 | 4.8 | 4.5 | 3.9 | 3.3 | 3.0 |
| Decanal freigesetzt aus Beispiel 4 | 5.7 | 6.0 | 6.2 | 6.0 | 5.0 | 4.6 | 4.3 |
| (6Z)-Non-6-enal freigesetzt aus Beispiel 26 | 5.2 | 4.7 | 4.0 | 3.8 | 3.7 | 3.9 | 3.3 |
| (5Z)-Oct-5-enal freigesetzt aus Beispiel 25 | 7.0 | 7.2 | 6.8 | 6.8 | 6.7 | 5.9 | 4.8 |

Die Fragrance Precursor in der Formulierung für Haarfärbung zeigten nach Vereinigung der Entwicklermasse mit der ammoniakalischen Haarfärbelösung eine nahezu spontane Hydrolyse zu den korrespondierenden Aldehyden. Bereits nach 4 Minuten haben die freigesetzten Aldehyde ihre maximale Intensität erreicht.

Hieraus ergibt sich überraschenderweise ein erheblicher Vorteil in der Verwendung der erfindungsgemäß als

Fragrance Precursor zu verwendenden Verbindungen der Formel (I) zur gezielten Freisetzung von Aldehyden oder Ketonen in Parfümölen für alkalische Haarfärbemittel.

**Beispiel 30 (nicht erfindungsgemäß): Seife**

Die folgende Seifenformulierung kann nach allgemein bekannten Methoden hergestellt werden. Die Angaben beziehen sich auf Gewichtsprozente. Die erhaltenen Seifen A und B wurden sowohl direkt als auch nach Lagerung für vier Wochen zum Waschen verwendet bzw. analytisch untersucht.

**Tabelle 4: Seifenformulierung**

| Inhaltsstoffe | | A | B |
|---|---|---|---|
| Seifenbase | Sodium Tallowate | 60,0 | 60,0 |
| Seifenbase | Sodium Cocoate | 27,0 | 27,0 |
| | Glycerine | 2,0 | 2,0 |
| | Sodium Chloride | 0,5 | 0,5 |
| Stabilisator | Tetrapotassium Etidronate | 0,3 | 0,3 |
| Stabilisator | Tocopherol | 0,1 | 0,1 |
| Färbemittel | Titanium Dioxide | 0,1 | 0,1 |
| | Water | 7,0 | 7,0 |
| | Diethylphtalat (DEP) | 2,4 | 2,4 |
| Beispiel 5 | (1E/Z)-Isobuttersäuredec-1-enylester | 0,60 | |
| | n-Decanal | | 0,60 |

(a) Geruchliche und farbliche Beurteilung:

Die Seifenformulierungen A und B wurden für ca. drei Monate bei Raumtemperatur gelagert.

Die erfindungsgemäße Seife A welche den Fragrance Precursor (1E/Z)-Isobuttersäuredec-1-enylester beinhaltet, zeigte farblich nicht oder nur geringfügige Veränderung, während die (Vergleichs-)Seife B eine gelbliche bzw. graue Verfärbung hatte. Durch die Verwendung des Fragrance Precursors wird somit eine hohe Farbstabilität erzielt.

Nach der Lagerung wurden jeweils 1g der Seifen in 100g handwarmen Wasser aufgelöst bzw. die Seifenstücke zum Waschen von Haut verwendet.

In allen Fällen war der Dufteindruck über der wässrigen Lösung der erfindungsgemäßen Seife A, welche den Fragrance Precursor beinhaltet, deutlich stärker als der Dufteindruck der Seife B, welche den freien Aldehyd beinhaltet.

Der Dufteindruck von der gewaschenen Haut, welche mit der Seife A gewaschen wurde, war ebenfalls höher als der Dufteindruck nach Waschen mit der Seife B.

(b) Lagerstabilität:

Die Seifenformulierungen A und B wurden für ca. einen Monat bei Raumtemperatur in der Dunkelheit gelagert. Das Depotpräparat in der erfindungsgemäßen Seife A zeigte eine deutlich höhere Lagerstabilität als der korrespondierende Aldehyd in (Vergleichs-)Seife B.

## Patentansprüche

**1.** Verfahren zur Freisetzung eines Riechstoffs, mit folgenden Schritten:

- Bereitstellen einer Verbindung der Formel I

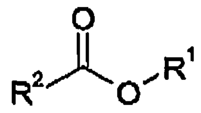

in der

$R^1$ der Rest (a) der Enolform eines Aldehyds mit 6 oder mehr C-Atomen oder (b) eines Ketons mit 10 oder mehr C-Atomen ist

und

$R^2$ eine (a) verzweigte oder unverzweigte $C_1$ bis $C_4$ Alkylgruppe oder (b) verzweigte oder unverzweigte $C_2$ bis $C_4$ Alkylengruppe ist,

- Herstellen einer Formulierung, die die Verbindung der Formel I und ein Medium umfasst, so dass die Verbindung der Formel I in der Formulierung stabil ist, wobei das Medium sauer und oxidativ ist und einen Wassergehalt ≤ 10 Gew.-%, bezogen auf die Gesamtmasse des Mediums, besitzt,
- Behandeln der Formulierung, so dass die Verbindung der Formel I zerfällt und den Riechstoff freisetzt, wobei die Behandlung der Formulierung umfasst, dass der pH-Wert der Formulierung auf einen Wert ≥ 8,5 angehoben wird,

**dadurch gekennzeichnet, dass** der freigesetzte Riechstoff ausgewählt wird aus der Liste bestehend aus:
Aldehyden: Phenylacetaldehyd, p-Methylphenylacetaldehyd, p-Isopropylphenylacetaldehyd, Methylnonyl acetaldehyd, phenylpropanal, 3-(4-t-Butylphenyl)-2-methylpropanal (Lilial), 3-(4-t-Butylphenyl)-propanal (Bourgeonal), 3-(4-Methoxyphenyl)-2-methylpropanal (Canthoxal). 3-(4- Isopropylphenyl)-2-methylpropanal (Cymal), 3-(3,4-Methylendioxyphenyl)-2-methylpropanal (Helional), 3-(4-Ethylphenyl)-2,2-dimethylpropanal (Floralozone), Phenylbutanal, 3-Methyl-5-phenylpentanal, Hexanal, trans-2-Hexenal, cis-Hex-3-enal, Hep-tanal, cis-4-Heptenal, 2-Ethyl-2-heptenal, 2,6-Dimethyl-5-heptenal (Melonal), 2,4-Heptadienal, Octanal, 2-Octenal, cis-5-Octenal, 3,7-Dimethyloctanal, 3,7-Dimethyl-2,6-octadien-1-al, 3,7-Dimethyl-2,6-octadien-3-al, 3,7-Dimethyl-6-octenal (Citronellal), 3,7-Dimethyl-7-hydroxyoctan-1-al (Hydroxy Citronellal), Nonanal, cis-6-Nonenal, 2,4-Nonadienal, 2,6-Nonadienal, Decanal, 2-Methyldecanal, 4-Decenal, 9-Decenal, 2,4-Decadienal, Undecanal, 2-Methyldecanal, 2-Methylundecanal, 2,6,10-Trimethyl-9-undecenal (Adoxal). Undec-10-enylaldehyd, Undec-8-enanal, Dodecanal, Tridecanal, Tetradecanal, Anisalde-hyd, Zimtaldehyd, a-Amylzimtaldehyd, a-Hexylzimtaldehyd, Methoxyzimtalde-hyd, Isocyclocitral, Citronellyloxyacetaldehyd, Cortexaldehyd, Cuminaldehyd, Cyclamenaldehyd, Florhydral, Heliotropin, Hydratropaaldehyd, Vanillin, Ethyl-vanillin, Benzaldehyd, p-Methylbenzaldehyd. 3,4-Dimethoxybenzaldehyd, 3- und 4-(4-Hydroxy-4-methylpentyl)-3-cyclohexen-1-carboxaldehyd (Lyral), 2,4-Dimethyl-3-cyclohexen-1-carboxaldehyd (Triplal), 1-Methyl-3-( 4-methylpentyl)-3-cyclohexencarboxaldehyd (Vernaldehyd) oder p-Methylphenoxyacetaldehyd (Xi aldehyd) oder
Ketonen: α-Damascon, β-Damascon, δ-Damascon, β-Damascenon, Muscon, 6,7-Dihydro-1,1,2,3,3-pentamethyl-4(5H)-indanon (Cashmeran), cis-Jasmon, Dihydrojasmon, α-Ionon, β-Ionon, Dihydro-β-ionon, g-Methylionon, α-iso-Methylionon, 4-(3,4-methylendioxyphenyl)butan-2-on, 4-(4-Hydroxyphenyl)butan-2-on, Methyl-β-naphthylketon, Methylcedrylketon, 6-Acetyl-1,1,2,4,4,7-hexamethyltetralin (Tonalid), I-Carvon, 5-Cyclohexadecen-1-on, Acetophenon, Decaton, p-Hydroxyphenylbutan-2-on, 2-[2-(4-Methyl-3-cyclohexenyl-1-yl)propyl]cyclopentan-2-on, 2-sec-Butylcyclohexanon. β-Dihydroionon, Allylionon, α-Iron, α-Ceton, α-Irison, Acetanisole, Geranylaceton, 1-(2-Methyl-5-isopropyl-2-cyclohexenyl)-1-propanon, Acetyldiisoamylen, Me-thylcyclocitron, 4-t-Pentylcyclohexanone, p-t-Butylcyclohexanon, o-t-Butylcyclohexanon, Ethylamylketon, Ethylpentylketon, Menthon, Methyl-7,3-Dlhydro-2H-1,5-benzodioxepin-3-on oder Fenchon.

**2.** Verfahren nach Anspruch 1, wobei die Formulierung ausgewählt ist aus der Gruppe bestehend aus: Entwicklermasse für permanentes Haarfärbemittel, Dauerwellenfixierung, Bleichcreme, Aknecreme, Sanitärreiniger und Oberflächenreiniger.

**3.** Kosmetische, Wasch- und/oder Reinigungs-Formulierung, umfassend oder bestehend aus:

- einer Verbindung der Formel I

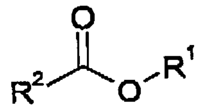

in der

$R^1$ der Rest (a) der Enolform eines Aldehyds mit 6 oder mehr C-Atomen oder (b) eines Ketons mit 10 oder mehr C-Atomen

und

$R^2$ eine (a) verzweigten oder unverzweigte $C_1$ bis $C_4$ Alkylgruppe oder (b) verzweigte oder unverzweigte $C_2$ bis $C_4$ Alkylengruppe ist

sowie
- einem Medium bestehend aus weiteren bzw. den weiteren Formulierungsbestandteilen, wobei das Medium sauer und oxidativ ist und einen Wassergehalt ≤ 10 Gew,-%, bezogen auf die Gesamtmasse des Mediums, besitzt,

wobei der Anteil der Verbindung der Formel I an der Formulierung geringer ist als 1 Gew.-%, bezogen auf die Gesamtmasse der Formulierung, und wobei das Medium so ausgewählt ist, dass die Verbindung der Formel I in der Formulierung stabil ist.

**4.** Formulierung nach Anspruch 3, wobei die Formulierung ausgewählt istaus der gruppe bestehend aus aus: Entwicklermasse für permanentes Haarfärbemittel, Dauerwellenfixierung, Bleichcreme, Aknecreme, Sanitärreiniger und Oberflächenreiniger.

**5.** Formulierung nach einem der Ansprüche 3 oder 4, wobei

(a) die Verbindung der Formel I in dem Medium dispergiert oder gelöst ist
und/oder
(b) die Verbindung der Formel I als Bestandteil eines Parfümöls eingesetzt ist, das in dem Medium dispergiert oder gelöst ist,

wobei im Falle (b) das Parfümöl gegebenenfalls (i) an einem Trägerstoff adsorbiert. (ii) mikroverkapselt oder (iii) sprühgetrocknet ist oder (iv) als Einschluss-Komplex oder (v) Extrusions-Produkt eingesetzt ist oder (vi) gecoatet ist.

**6.** (1E/Z)-Isobuttersäure-2,6-dimethylhepta-1,5-dienylester.

## Claims

**1.** Method for release of an odoriferous substance with the following steps:

- provision of a compound of the formula I

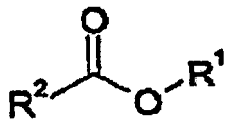

in which

$R^1$ is the radical (a) of the enol form of an aldehyde having 6 or more C atoms or (b) of a ketone having 10 or more C atoms

and

$R^2$ is a (a) branched or unbranched $C_1$ to $C_4$ alkyl group or (b) branched or unbranched $C_2$ to $C_4$ alkylene group,

- preparation of a formulation which comprises the compound of the formula I and a medium such that the compound of the formula I is stable in the formulation, wherein the medium is acidic and oxidative and has a water content of ≤ 10 wt.%, based on the total weight of the medium,
- treatment of the formulation such that the compound of the formula I dissociates and releases the odoriferous substance, wherein the treatment of the formulation comprises raising the pH of the formulation to a value of ≥ 8.5,

**characterized in that** the odoriferous substance released is chosen from the list consisting of:

aldehydes: phenylacetaldehyde, p-methylphenylacetaldehyde, p-isopropylphenylacetaldehyde, methylnonylacetaldehyde, phenylpropanal, 3-(4-t-butylphenyl)-2-methylpropanal (lilial), 3-(4-t-butylphenyl)-propanal (bourgeonal), 3-(4-methoxyphenyl)-2-methylpropanal (canthoxal), 3-(4-isopropylphenyl)-2-methylpropanal (cymal), 3-(3,4-methylenedioxyphenyl)-2-methylpropanal (helional), 3-(4-ethylphenyl)-2,2-dimethylpropanal (floralozone), phenylbutanal, 3-methyl-5-phenylpentanal, hexanal, trans-2-hexenal, cis-hex-3-enal, heptanal, cis-4-heptenal, 2-ethyl-2-heptenal, 2,6-dimethyl-5-heptenal (melonal), 2,4-heptadienal, octanal, 2-octenal, cis-5-octenal, 3,7-dimethyloctanal, 3,7-dimethyl-2,6-octadien-1-al, 3,7-dimethyl-2,6-octadien-3-al, 3,7-dimethyl-6-octenal (citronellal), 3,7-dimethyl-7-hydroxyoctan-1-al (hydroxycitronellal), nonanal, cis-6-nonenal, 2,4-nonadienal, 2,6-nonadieenal, decanal, 2-methyldecanal, 4-decenal, 9-decenal, 2,4-decadienal, undecanal, 2-methyldecanal, 2-methylundecanal, 2,6,10-trimethyl-9-undecenal (adoxal), undec-10-enylaldehyde, undec-8-enanal, dodecanal, tridecanal, tetradecanal, anisaldehyde, cinnamaldehyde, α-amylcinnamaldehyde, α-hexylcinnamaldehyde, methoxycinnamaldehyde, isocyclocitral, citronellyloxyacetaldehyde, cortexaldehyde, cuminaldehyde, cyclamenaldehyde, florhydral, heliotropin, hydratropaaldehyde, vanillin, ethylvanillin, benzaldehyde, p-methylbenzaldehyde, 3,4-dimethoxybenzaldehyde, 3- and 4-(4-hydroxy-4-methylpentyl)-3-cyclohexene-1-carboxaldehyde (lyral), 2,4-dimethyl-3-cyclohexene-1-carboxaldehyde (tripial), 1-methyl-3-(4-methylpentyl)-3-cyclohexenecarboxaldehyde (vernaldehyde) or p-methylphenoxyacetaldehyde (xi aldehyde) or
ketones: α-damascone, β-damascone, δ-damascone, p-damascenone, muscone, 6,7-dihydro-1,1,2,3,3-pentamethyl-4(5H)-indanone (cashmeran), cis-jasmone, dihydrojasmone, α-ionone, β-ionone, dihydro-β-ionone, γ-methylionone, α-isomethylionone, 4-(3,4-methylenedioxyphenyl)butan-2-one, 4-(4-hydroxyphenyl)butan-2-one, methyl β-naphthyl ketone, methyl cedryl ketone, 6-acetyl-1,1,2,4,4,7-hexamethyltetralin (tonalid), 1-carvone, 5-cyclohexadecen-1-one, acetophenone, decatone, p-hydroxyphenylbutan-2-one, 2-[2-(4-methyl-3-cyclohexenyl-1-yl)propyl]cyclopentan-2-one, 2-sec-butylcyclohexanone, β-dihydroionone, allylionone, α-irone, α-cetone, α-irisone, acetanisoles, geranyl acetone, 1-(2-methyl-5-isopropyl-2-cyclohexenyl)-1-propanone, acetyldiisoamylene, methylcyclocitrone, 4-t-pentyl cyclohexanone, p-t-butylcyclohexanone, o-t-butylcyclohexanone, ethyl amyl ketone, ethyl pentyl ketone, menthone, methyl-7,3-dihydro-2H-1,5-benzodioxepin-3-one or fenchone.

2. Method according to claim 1, wherein the formulation is chosen from the group consisting of: developer compositions for permanent hair colouring compositions, permanent wave fixing agents, bleaching creams, acne creams, sanitaryware cleaners and surface cleaners.

3. Cosmetic, washing and/or cleaning formulation, comprising or consisting of:

- a compound of the formula I

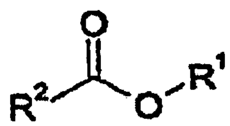

in which

$R^1$ is the radical (a) of the enol form of an aldehyde having 6 or more C atoms or (b) of a ketone having 10 or more C atoms

and

$R^2$ is a (a) branched or unbranched $C_1$ to $C_4$ alkyl group or (b) branched or unbranched $C_2$ to $C_4$ alkylene group,

and
- a medium comprising further or the further formulation constituents, wherein the medium is acidic and oxidative and has a water content of s 10 wt.%, based on the total weight of the medium,

wherein the content of the compound of the formula I in the formulation is less than 1 wt.%, based on the total weight of the formulation, and wherein the medium is chosen such that the compound of the formula I is stable in the formulation.

**4.** Formulation according to claim 3, wherein the formulation is chosen from the group consisting of developer compositions for permanent hair colouring compositions, permanent wave fixing agents, bleaching creams, acne creams, sanitaryware cleaners and surface cleaners.

**5.** Formulation according to one of claims 3 or 4, wherein

(a) the compound of the formula I is dispersed and/or dissolved in the medium
and/or
(b) the compound of the formula I is employed as a constituent of a perfume oil dispersed or dissolved in the medium,

wherein in case (b) the perfume oil optionally is (i) adsorbed on a carrier substance, (ii) microencapsulated or (iii) spray-dried or is employed (iv) as an inclusion complex or (v) extruded product or (vi) is coated.

**6.** (1E/Z)-Isobutyric acid 2,6-dimethylhepta-1,5-dienyl ester.

**Revendications**

**1.** Procédé pour dégager une matière odorante, avec les étapes suivantes :

- mise à disposition d'un composé de la formule I

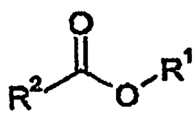

dans laquelle

$R^1$ est le radical (a) de la forme énolique d'un aldéhyde à 6 atomes de carbone ou plus ou (b) d'une cétone à 10 atomes de carbone ou plus
et
$R^2$ est (a) un groupe alkyle ramifié ou linéaire en $C_1$ à $C_4$ ou (b) un groupe alkylène ramifié ou linéaire en $C_2$ à $C_4$,

- préparation d'une formulation qui comporte le composé de la formule I et un milieu, de telle sorte que dans la formulation, le composé de la formule I soit stable, le milieu étant acide et oxydant et possédant une teneur en eau de ≤10 % en poids, relative à la masse totale du milieu,
- traitement de la formulation, de telle sorte que le composé de la formule I se décompose et libère la substance odorante, le traitement de la formulation comprenant que le pH de la formulation soit relevé à une valeur ≥8,5,

**caractérisé en ce que** la substance odorante libérée est choisie dans la liste consistant en :

les aldéhydes : phénylacétaldéhyde, p-méthylphénylacétaldéhyde, p-isopropylphénylacétaldéhyde, méthylnonylacétaldéhyde, phénylpropanal, 3-(4-t-butylphényle)-2-méthylpropanal (Lilial), 3-(4-t-butylphényle)-propanal (Bourgeonal), 3-(4-méthoxyphényl)-2-méthylpropanal (Canthoxal), 3-(4-isopropylphényl)-2-méthylpropanal (Cymal), 3-(3,4-méthylènedioxyphényl)-2-méthylpropanal (Hélional), 3-(4-éthylphényl)-2,2-diméthylpropanal (Floralozone), phénylbutanal, 3-méthyl-5-phénylpentanal, hexanal, trans-2-hexénal, cis-hex-3-énal, heptanal, cis-4-hepténal, 2-éthyle-2-hepténal, 2,6-diméthyl-5-hepténal (Mélonal), 2,4-heptadiénal, octanal, 2-octénal, cis-5-octénal, 3,7-diméthyloctanal, 3,7-diméthyl-2,6-octadiène-1-al, 3,7-diméthyl-2,6-octadiène-3-al, 3,7-diméthyl-6-octénal (Citronellal), 3,7-diméthyl-7-hydroxyoctane-1-al (Hydroxy Citronellal), nonanal, cis-6-nonénal, 2,4-nonadiénal, 2,6-nonadiénal, décanal, 2-méthyldécanal, 4-décénal, 9-décénal, 2,4-décadiénal, undécanal, 2-méthyldécanal, 2-méthylundécanal, 2,6,10-triméthyle-9-undécénal (Adoxal), undéc-10-énylaldéhyde, undéc-8-ènanal, dodécanal, tridécanal, tétradécanal, anisaldéhyde, cinnamaldéhyde, a-amyl-cinnamaldéhyde, a-hexylcinnamaldéhyde, méthoxycinnamaldéhyde, isocyclocitral, citronellyloxyacétaldéhyde, cortexaldéhyde, cuminaldéhyde, aldéhyde cyclaménique, florhydral, héliotropine, hydratropaaldéhyde, acide vanillique, éthylevanilline, benzaldéhyde, p-méthylbenzaldéhyde, 3,4-diméthoxybenzaldéhyde, 3- et 4-(4-hydroxy-4-méthylpentyl)-3-cyclohexène-1-carboxaldéhyde (Lyral), 2,4-diméthyl-3-cyclohexène-1-carboxaldéhyde (Triplal), 1-méthyl-3-(4-méthylpentyl)-3-cyclohexèncarboxyaldéhyde (Vernaldéhyde) ou p-méthylphénoxyacétaldéhyde (aldéhyde Xi) ou

les cétones : α-damascone, β-damascone, δ-damascone, β-damascenone, muscone, 6,7-dihydro-1,1,2,3,3-pentaméthyl-4(5H)-indanone (Cashmeran), cis-jasmone, dihydrojasmone, α-ionone, β-ionone, dihydro-β-ionone, g-méthylionone, α-iso-méthylionone, 4-(3,4-méthylèndioxyphényl)butan-2-one, 4-(4-hydroxyphényl)butan-2-one, méthyl-β-naphthylcétone, méthylcédrylcétone, 6-acétyl-1,1,2,4,4,7-hexaméthyltétraline (Tonalid), 1-carvone, 5-cyclohexadécén-1-one, acétophénone, décatone, p-hydroxyphénylbutan-2-one, 2-[2-(4-méthyl-3-cyclohexényl-1-yl)propyl]cyclopentan-2-one, 2-sec-butylcyclohexanone, β-dihydroionone, allylionone, α-irone, α-cétone, α-irisone, acétanisole, géranylacétone, 1-(2-méthyl-5-isopropyl-2-cyclohexényl)-1-propanone, acétyldiisoamylène, méthylcyclocitrone, 4-t-pentylcyclohexanone, p-t-butylcyclohexanone, o-t-butylcyclohexanone, éthylamylcétone, éthylpentylcétone, menthone, méthyl-7,3-dihydro-2H-1,5-benzodioxépin-3-one ou fenchone.

2. Procédé selon la revendication 1, la formulation étant choisie parmi le groupe constitué de : masse de révélateur pour teinture de cheveux permanente, fixation pour mise en plis permanente, crème éclaircissante, crème contre l'acné, nettoyant sanitaire et nettoyant de surfaces.

3. Formulation cosmétique de lavage et/ou de nettoyage, comportant ou constituée de :

- un composé de la formule 1

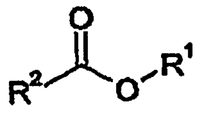

dans laquelle

$R^1$ est le radical (a) de la forme énolique d'un aldéhyde à 6 atomes de carbone ou plus ou (b) d'une cétone à 10 atomes de carbone ou plus
et
$R^2$ est (a) un groupe alkyle ramifié ou linéaire en $C_1$ à $C_4$ ou (b) un groupe alcényle ramifié ou linéaire en $C_2$ à $C_4$,

ainsi que

- d'un milieu constitué d'autres, respectivement des autres composants de la formulation, le milieu étant acide et oxydant et possédant une teneur en eau de ≤10 % en poids, rapportée à la masse totale du milieu,

la proportion du composé de la formule I dans la formulation étant inférieure à 1 % en poids, rapporté à la masse totale de la formulation et le milieu étant choisi de telle sorte que le composé de la formule I soit stable dans la formulation.

**4.** Formulation selon la revendication 3, la formulation étant choisie parmi le groupe constitué de : masse de révélateur pour teinture de cheveux permanente, fixation pour mise en plis permanente, crème éclaircissante, crème contre l'acné, nettoyant sanitaire et nettoyant de surfaces.

**5.** Formulation selon l'une quelconque des revendications 3 ou 4, dans laquelle

(a) le composé de la formule I est dispersé ou dissout dans le milieu
et/ou
(b) le composé de la formule I est utilisé comme composant d'une huile de parfum qui est dispersée ou dissoute dans le milieu,

sachant que dans le cas (b), l'huile de parfum est, le cas échéant, (i) adsorbée sur une substance support, (ii) micro-encapsulée ou (iii) séchée par pulvérisation, ou (iv) est utilisée comme complexe d'insertion ou (v) comme produit d'extrusion, ou (vi) est fixée par enrobage.

**6.** (1E/Z)-acide isobutyrique-2,6-diméthylhepta-1,5-diénylester.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5649979 A
- US 6207857 B
- US 6479682 B
- US 6262287 B
- US 3923247 A
- JP 5514137 A
- JP 55015433 B
- US 3023247 A


### In der Beschreibung aufgeführte Nicht-Patentliteratur


- **Gerasimovich. T.B. et al.** *Natural'nykh Dushistykh Veshchestv,* 1965, 38-42
- **S. Arctander.** Perfume and Flavor Materials. Selbstverlag, 1969, vol. I, II
- **K. Bauer ; D. Garbe ; H, Surburg.** Common Fragrance and Flavor Materials. Wiley-VCH, 1997
- **D.P. Simmons et al.** *Helv. Chim. Acta,* 1988, vol. 71, 1000
- **P. Duhamel et al.** *J. Chem. Soc. Perkin Trans. 1,* 1993, 2509
- **P.Z, Bedoukian.** *J. Am. Chem. Soc.,* 1957, vol. 79, 889-892
- **P.Z. Bedoukian.** *J. Am. Chem. Soc.,* 1957, vol. 79, 889-892
- **B.M. Trost et al.** *J. Am. Chem. Soc.,* 1978, vol. 100, 3930-3931